# EUROPEAN PATENT SPECIFICATION

(11) **EP 0 675 874 B1**
(45) Date of publication and mention of the grant of the patent: **19.03.1997**
(21) Application number: 94903131.4
(22) Date of filing: 26.11.1993
(51) Int. Cl.: C07C 253/26, C07C 253/24, B01J 23/31, B01J 23/88

(54) **PROCESS FOR THE PREPARATION OF (METH)ACRYLONITRILE BY AMMOXIDATION**
PROZESS ZUR HERSTELLUNG VON (METH)ACRYLNITRIL DURCH AMMOXIDATION
PROCEDE DE PREPARATION DE (METH)ACRYLONITRILE PAR AMMOXYDATION

(30) Priority: 23.12.1992 BE 9201131
(43) Date of publication of application: 11.10.1995
(73) Proprietor: DSM N.V., 6411 TE Heerlen (NL)
(72) Inventor: VAN DEN BRINK, Franciscus Tobias Bernardus Joseph, NL-5663 AE Geldrop (NL); VAN WIJCK, Julius, Gerardus, Theodorus, Nl-6211 SL Maastricht (NL)
(74) Representative: Jacobs, Monique S.N.
(86) International application number: PCT/NL93/00253
(87) International publication number: WO 94/14759

(56) References cited:
- EP-A- 0 344 884
- EP-A- 0 437 056
- DE-A- 1 468 669
- NL-A- 7 017 149
- US-A- 4 788 173
- US-A- 4 855 275
- CHEMIE-INGENIEUR-TECHNIK vol. 38, no. 7 , 1966 pages 701 - 704 R. SCHÖNBECK ET AL. 'Acrylnitril aus Propylen, Ammoniak und Luftsauerstoff'

## Description

The invention relates to a process for the preparation of (meth)acrylonitrile by ammoxidation of a C₃₋₄ alkane and alkene mixture.

A similar process is known from US-A-4788173, in which it is described that a mixture of for instance propane and minor quantities of propene can be used as feed for the reaction yielding acrylonitrile.

The drawback of the process as described in US-A-4788173 is that the conversion is low when mainly propane is used as feed. Consequently larger gas flows are required to obtain an equally large capacity compared with propene feeds. Moreover, when propane is used as feed, relatively much heat is generated. This implies that a large reactor is required with a large heat-exchanging surface area. Propane is indeed a cheaper raw material than propene - which for many years was used as a raw material in the manufacture of acrylonitrile - but an existing production facility will have to be fully replaced if propane is to be used instead of propene. This is not only necessary because of the larger reactor volume, but also because of the (economic) necessity to use the additionally generated energy. Turbines or generators, for instance, will be required then.

Another drawback of the use of propane, compared with propene, is that the acrylonitrile yield is relatively low. A further drawback is that propene, a valuable raw material is produced, which cannot be fully utilized.

The object of the invention is to eliminate the above-mentioned drawbacks.

This object is achieved by using a mixture of 50-98% alkene and 2-50% alkane, the ammoxidation being effected by applying a catalyst composition which converts both alkane and alkene.

By % is meant % by volume in this application, unless specified otherwise.

As C₃₋₄ alkane and alkene is used either the combination propane/propene or the combination isobutane/isobutene, to obtain acrylonitrile or (meth)acrylonitrile, respectively. Preferably, the combination propane/propene is used.

By the invention it is achieved that the (meth)acrylonitrile yield remains virtually the same and that the same installations can be used, while propane or isobutane can be utilized in substantial quantities.

By preference, a mixing ratio of 65-96% alkene and 4-35% alkane is used.

According to a first preferred embodiment there is advantage in applying the process according to the invention with chemical-grade propene containing about 4% propane. In that way this raw material is utilized optimally.

In a second preferred embodiment the process according to the invention is applied with 85-95% alkene and 15-5% alkane because in that case the alkane can be utilized optimally and (virtually) no investments are required in existing (meth)acrylonitrile plants.

US-A-3280166 describes that together with an olefin as raw material for the ammoxidation a corresponding saturated compound can be used as (inert) dilution gas. There is no indication that the saturated alkanes are converted.

The preparation of acrylonitrile for instance is based on the catalytic ammoxidation of for instance propene with ammonia and oxygen (air) in accordance with the following reaction equation:

CH₂=CH-CH₃ + NH₃ + 3/2 O₂ → CH₂=CH-CN + 3 H₂O

This reaction takes place in a fluid-bed reactor at temperatures varying between 350 and 700°C. The temperature is mostly between 400 and 550°C. The reaction pressure is as a rule between 1 and 3 atmosphere.

The C₃₋₄ alkene/alkane mixture is contacted in the reaction zone in the gas phase with ammonia, molecular oxygen and, optionally, with an inert dilution agent and a catalyst.

The molar ratio NH₃ : alkane/alkene is mostly between 0.2 and 20, preferably between 0.6 and 10. The molar ratio O₂ : alkene/alkane mostly varies from 1 to 10, preferably from 1.5 to 5.

In the process according to the invention a catalyst composition, which converts both alkane and alkene, is used.

The catalyst composition preferably has such an activity under reaction conditions that more than 80% alkene and more than 10% alkane is converted. It is particularly preferred for the activity to be such that more than 90% alkene and more than 50% alkane is converted.

A catalyst composition that can be used to convert both C₃₋₄ alkane and alkene can consist of a homogeneous composition or of a physical mixture of different, preferably two, catalysts. It is possible to define different reactions.

It is possible to use a mixture of catalysts, the mixture consisting of a first catalyst converting alkane into alkene and a second catalyst converting alkene into (meth)-acrylonitrile. These catalysts are described in US-A-4788173, US-A-4783545, US-A-4767739, US-A-4769355, US-A-4814478, US-A-4801727, US-A-4888438, US-A-4866195, US-A-4837191, US-A-4843055, US-A-4897504, US-A-4835125, US-A-4874738, US-A-4912214, US-A-4866024 and US-A-4883895. This catalyst composition can be composed as follows: A first catalyst consists for 10-99 wt.% of a thinner/carrier and 90-1 wt.% of a compound which has the following general formula

VSbₘAₐB_{b}C_{c}TₜOₓ (1)

where
A can consist of one or more elements, which may be the same or different, chosen from the group comprising W, Sn, Mo, B, P and Ge,
B can consist of one or more elements, which may be the same or different, chosen from the group comprising Fe, Co, Ni, Cr, Pb, Mn, Zn, Se, Te, Ga, In and As,
C can consist of one or more elements, which may be the same or different, chosen from the group comprising alkali metals and Tl,
T can consist of one or more elements, which may be the same or different, chosen from the group comprising Ca, Sr and Ba,
1<m<20, 0≤a≤10, 0≤b≤20, 0≤c≤20, 0≤t≤20, a≤m, b≤m, c≤m, t≤m and x is determined by the degree of oxidation of the other elements, Sb has an average valency higher than +3 and V has an average valency lower than +5. The second catalyst consists for 0-99 wt.% of a thinner/carrier and 100-1 wt.% of a compound which answers the following general formula

   BiₙCeₚD_{d}EₑF_{f}Mo₁₂W_{g}O_{y} (2)

   where
D can consist of one or more elements, which may be the same or different, chosen from the group comprising Fe, Mn, Pb, Co, Ni, Cu, Sn, P, Cr, Y, Mg, Ca, Sr and Ba,
E can consist of one or more elements, which may be the same or different, chosen from the group comprising Sb, Ge, As, Se, Te and V,
F can consist of one or more elements, which may be the same or different, chosen from the group comprising alkali metals, Tl, Ag and Sm,
0.01≤n≤24, 0.01≤p≤24, 0.1≤n+p≤24, 0≤d≤10, 0≤e≤10, 0≤f<6, 0≤g≤8 and y is determined by the degree of oxidation of the other elements.

Normally, the carrier or the dispersant of compound (1) is not an oxide of an element of compound (1); the same applies to compound (2). For the preparation of these catalysts, see the above-mentioned patents.

These catalysts may also show ammoxidation activity with respect to propane.

It is also possible to use a catalyst which converts for instance both propene and propane into acrylonitrile. Such a catalyst is described in US-A-4760159 and EP-A-282314 and answers the general formula

BiₕVᵥLₗMₖT_{q}Oᵣ (3)

where
L can consist of one or more elements, which may be the same or different, chosen from the group comprising K, Cs, Rb and Tl,
M can consist of one or more elements, which may be the same or different, chosen from the group comprising Mo, W, Cr, Ge or Sb,
T can consist of one or more elements, which may be the same or different, chosen from the group comprising Ce, Zn, B, Nb and Ta,
h = 1-25, v = 1-50, 1 = 0-1, preferably 0-0.2, k = 0.1-20, q = 0-20 and r is determined by the degree of oxidation of the other elements, (h+v):(l+k+q) = 20:1-1:5, h:v = 1:5-5:1, preferably 1:3-3:1.

This catalyst can be used as such, but mostly contains an inorganic oxide as carrier or thinner.

Catalysts can also consist of a physical mixture of a catalyst that is specific for the propene ammoxidation and one for the propane ammoxidation. Generally, in that case less organic oxide is required as carrier/thinner.

It is also possible to use a mixture of the different types of catalysts, since the reactions that are possible with the different types of catalysts proceed up to a certain level.

Catalysts for the ammoxidation of C₃₋₄ alkane are described for instance in US-A-4736054, US-A-4746641, US-A-4788317, US-A-4873215, US-A-4797381 and US-A-4871706. Preferably, a catalyst as described in US-A-4788317 is used.

Catalysts for the ammoxidation of alkene are described for instance in US-A-4495109, US-A-4659689, US-A-4724135, US-A-4766102 and US-A-4801727.

A very suitable catalyst for the ammoxidation of C₃₋₄ alkene is described for instance in US-A-4855275 and answers the general formula

MeₐSb_{b}X_{c}R_{d}QₑO_{f} (4)

where
Me can consist of one or more elements, which may be the same or different, chosen from the group comprising Fe, Co, Ta, Ni, Mn, U, Ce, Sn and Cu,
X can consist of one or more elements, which may be the same or different, chosen from the group comprising V, Mo, W, Nb and Ta,
R can consist of one or more elements, which may be the same or different, chosen from the group comprising B, P, Bi and Te,
Q can consist of one or more elements, which may be the same or different, chosen from the group comprising Mg, Ca, Sr, Ba, La, Ti, Zr, Cr, Re, Ru, Os, Rh, Ir, Pd, Pt, Ag, Cd, Al, Ga, In, Tl, Si, B, Ge, As and Se,
a = 10, b = 5-50, c = 0-30, d = 0-10, e = 0-20 and f is determined by the degree of oxidation of the other elements.

The catalysts that can be applied in the process according to the invention in general have a composition according to formulas (1) - (4). It is therefore preferred to use one or more catalysts having a composition according to formula(s) (1), (2), (3) and/or (4).

Preferably, use is made of physical mixtures of catalysts. The mixing ratio of the catalysts can be varied depending on the alkene:alkane ratio of the feed. The amount of each type of catalyst in the mixture will as a rule be between 40:60 and 98:2 by weight of catalyst for the conversion of alkene and the conversion of alkane.

The invention will be further illustrated by means of the following examples.

### Example I

A first catalyst (catalyst I) was prepared in a solution of 89 g of ammonium paratungstate, 73 g of ammonium metavanadate was dissolved at 55°C. Next, 406 g of ammonium paramolybdate was dissolved at 35°C in the resulting solution.

In a second vessel, 363 kg of 70 wt.% HNO₃ was added to an aqueous solution of metal nitrates (4.3wt.% Fe, 1.5 wt.% Cu, 1.3 wt.% Te and 23.7 wt.% NO₃⁻).

A vessel equiped with a reflux condenser and a stirrer was filled with 32 kg of water and 32 kg of 40% silicasol (ammonium-stabilized) while stirring. Next, the contents of the second vessel were added as well as, after stirring for 5 minutes, 12 kg of Sb₂O₃. Then the first solution was added. After stirring for 5 minutes a 15 wt.% aqueous ammonia solution was added in portions, so that the temperature of the reaction mixture remained around 35°C and a pH of 2.35 (at 40°C) was obtained.

The slurry was heated up to the reflux temperature and kept at reflux temperature for 4 hours. The slurry was removed and dry sprayed. The dried material was heated for 3 hours at 290°C, for 3 hours at 425°C, ground and calcined at 825°C for 3 hours.

Further, a catalyst (catalyst II) which is in particular suitable for the ammoxidation of propane and in addition yielded some propene, was prepared.

Bismuth nitrate dissolved in diluted nitric acid was mixed with a solution of ammonium metavanadate, ammonium heptamolybdate in hot water, and with chrome nitrate dissolved in water. Silicasol and aluminasol were added to the solution and the resulting slurry was dried. The dry material was heated for 3 hours at 290°C, for 3 hours at 425°C and for 3 hours at 550°C. The composition of this catalyst component was 50% BiV_{0.7}Mo_{0.5}Cr_{0.5}Oₓ + 40% SiO₂ + 10% Al₂O₃.

A 2 cm (internal diameter) glass reactor, provided with a thermocouple, was filled with a mixture of catalysts I and II (total 40 g, the ratio of catalyst I:II was 2:1). Next, a gas flow of the following composition was supplied - through a glass filter - to the reactor: 1.7 Nl/h (propane:propene = 30:70 v/v); 3.9 Nl/h oxygen; 14.8 Nl/h nitrogen and 1.8 Nl/h ammonia. The reaction products were analyzed by means of gas chromatography. The expanded bed height of the catalyst in the fluidized bed was about 32 cm, so that the density of the catalyst bed was about 400 kg/m³. At a reactor temperature of 470°C the propene conversion was 95% and the propane conversion 67%. The yield of acrylonitrile, HCN, CO₂ and CO on a molar basis with respect to propene and propane was 63.9%, 4.8%, 10.7% and 4.7%, respectively. On the basis of these data, a productivity of 25.6 kg of ACN per cubic meter of reactor capacity per hour at an energy production of 0.49 GJ/m³/h were calculated.

### Comparative experiment A

The ammoxidation experiment of example I was repeated, using only catalyst I and pure propene. The propene conversion was 95%. The productivity of the reactor was 29.9 kg of ACN/m³/h at an energy production of 0.51 GJ/m³/h.

By comparing Example I with comparative Example A, it appears that when mixtures of propene and propane are used at a certain energy production per volume unit, a high acrylonitrile yield can be achieved, which is close to the value that is obtained with pure propene.

### Example II

Example I was repeated, with 20% less silica being used for the preparation of catalyst I and 10% less for catalyst II. Under otherwise comparable conditions a productivity of 27.5 kg of ACN/m³/h was achieved at an energy production of 0.53 GJ/m³/h.

### Comparative experiment B

The ammoxidation experiment of example I was repeated, using only catalyst II and pure propane. The propane conversion was 67%, and 40% ACN, 2.7% HCN and 6% propene were formed. The productivity was 15.5 kg of ACN/m³/h at an energy production of 0.45 GJ/m³/h.

By comparing example II with comparative experiment B it appears that when mixtures of propene and propane are used at a certain energy production per volume unit, a high acrylonitrile yield can be achieved, which is close to the value that is obtained with pure propane.

## Claims

1. Process for the preparation of (meth)acrylonitrile by ammoxidation of a C₃₋₄ alkane and alkene mixture, characterized in that a mixture of 50-98% alkene and 2-50% alkane is used and the ammoxidation is effected by applying a catalyst composition which converts both alkane and alkene.

2. Process according to claim 1, characterized in that the mixing ratio is 65-96% alkene and 4-35% alkane.

3. Process according to any one of claims 1-2, characterized in that the mixing ratio is 85-95% alkene and 15-5% alkane.

4. Process according to any one of claims 1-3, characterized in that a mixture of propene and propane is used.

5. Process according to claim 3, characterized in that a mixture is applied with about 4% propane and about 96% propene.

6. Process according to any one of claims 1-5, characterized in that a catalyst composition is used which converts more than 80% of the alkene and more than 10% of the alkane.

7. Process according to claim 6, characterized in that a catalyst composition is used which converts more than 90% of the alkene and more than 50% of the alkane.

8. Process according to any one of claims 1-7, characterized in that a catalyst composition of two catalysts is used for the ammoxidation.

9. Process according to any one of claims 1-8, characterized in that the catalyst composition comprises compounds having the following general formula(s) (1), (2), (3) and/or (4):
VSbₘAₐB_{b}C_{c}TₜOₓ (1)
where
A can consist of one or more elements, which may be the same or ditferent, chosen from the group comprising W, Sn, Mo, B, P and Ge,
B can consist of one or more elements, which may be the same or different, chosen from the group comprising Fe, Co, Ni, Cr, Pb, Mn, Zn, Se, Te, Ga, In and As,
C can consist of one or more elements, which may be the same or different, chosen from the group comprising alkali metals and Tl,
T can consist of one or more elements, which may be the same or different, chosen from the group comprising Ca, Sr and Ba,
1<m<20, 0≤a≤10, 0≤b≤20, 0≤c≤20, 0≤t≤20, a≤m, b≤m, c≤m, t≤m and x is determined by the degree of oxidation of the other elements, Sb has an average valency higher than +3 and V has an average valency lower than +5;
BiₙCeₚD_{d}EₑF_{f}MO₁₂W_{g}O_{y} (2)
where
D can consist of one or more elements, which may be the same or different, chosen from the group comprising Fe, Mn, Pb, Co, Ni, Cu, Sn, P, Cr, Y, Mg, Ca, Sr and Ba,
E can consist of one or more elements, which may be the same or different, chosen from the group comprising Sb, Ge, As, Se, Te and V,
F can consist of one or more elements, which may be the same or different, chosen from the group comprising alkali metals, Tl, Ag and Sm,
0.01≤n≤24, 0.01≤p≤24, 0.1≤n+p≤24, 0≤d≤10, 0≤e≤10, 0≤f≤6, 0≤g≤8 and y is determined by the degree of oxidation of the other elements;
BiₕVᵥLₗMₖT_{q}Oᵣ (3)
where
L can consist of one or more elements, which may be the same or different, chosen from the group comprising K, Cs, Rb and Tl,
M can consist of one or more elements, which may be the same or different, chosen from the group comprising Mo, W, Cr, Ge or Sb,
T can consist of one or more elements, which may be the same or different, chosen from the group comprising Ce, Zn, B, Nb and Ta,
h = 1-25, v = 1-50, 1 = 0-1, preferably 0-0.2, k = 0.1-20, q = 0-20 and r is determined by the degree of oxidation of the other elements, (h+v):(l+k+q) = 20:1-1:5, h:v = 1:5-5:1, preferably 1:3-3:1;
MeₐSb_{b}X_{c}R_{d}QₑO_{f} (4)
where
Me can consist of one or more elements, which may be the same or different, chosen from the group comprising Fe, Co, Ta, Ni, Mn, U, Ce, Sn and Cu,
X can consist of one or more elements, which may be the same or different, chosen from the group comprising V, Mo, W, Nb and Ta,
R can consist of one or more elements, which may be the same or different, chosen from the group comprising B, P, Bi and Te,
Q can consist of one or more elements, which may be the same or different, chosen from the group comprising Mg, Ca, Sr, Ba, La, Ti, Zr, Cr, Re, Ru, Os, Rh, Ir, Pd, Pt, Ag, Cd, Al, Ga, In, Tl, Si, B, Ge, As and Se,
a = 10, b = 5-50, c = 0-30, d = 0-10, e = 0-20 and f is determined by the degree of oxidation of the other elements.

## Patentansprüche

1. Verfahren zur Herstellung von (Meth)acrylonitril durch Ammoxidation von einem C₃₋₄-Alkan- und -Alkengemisch, dadurch gekennzeichnet, daß ein Gemisch von 50-98% Alken und 2-50% Alkan verwendet und die Ammoxidation bewirkt wird durch Anwendung einer Katalysatorzusammensetzung, die sowohl Alkan als auch Alken umwandelt.

2. Verfahren nach Anspruch 1, dadurch gekennzeichnet, daß das Mischungsverhältnis 65-96% Alken und 4-35% Alkan beträgt.

3. Verfahren nach einem der Ansprüche 1 bis 2, dadurch gekennzeichnet, daß das Mischungsverhältnis 85-95% Alken und 15-5% Alkan beträgt.

4. Verfahren nach einem der Ansprüche 1 bis 3, dadurch gekennzeichnet, daß ein Gemisch aus Propen und Propan verwendet wird.

5. Verfahren nach Anspruch 3, dadurch gekennzeichnet, daß ein Gemisch mit etwa 4% Propan und etwa 96% Propen angewandt wird.

6. Verfahren nach einem der Ansprüche 1 bis 5, dadurch gekennzeichnet, daß eine Katalysatorzusammensetzung verwendet wird, die mehr als 80% des Alkens und mehr als 10% des Alkans umwandelt.

7. Verfahren nach Anspruch 6, dadurch gekennzeichnet, daß eine Katalysatorzusammensetzung verwendet wird, die mehr als 90% des Alkens und mehr als 50% des Alkans umwandelt.

8. Verfahren nach einem der Ansprüche 1 bis 7, dadurch gekennzeichnet, daß eine Katalysatorzusammensetzung aus zwei Katalysatoren für die Ammoxidation verwendet wird.

9. Verfahren nach einem der Ansprüche 1 bis 8, dadurch gekennzeichnet, daß die Katalysatorzusammensetzung Verbindungen der folgenden allgemeinen Formel oder Formeln (1), (2), (3) und/oder (4) umfaßt:
VSbₘAₐB_{b}C_{c}TₜOₓ (1)
worin
A aus einem oder mehreren Elementen bestehen kann, die gleich oder verschieden sein können, ausgewählt aus der Gruppe bestehend aus W, Sn, Mo, B, P und Ge,
B aus einem oder mehreren Elementen bestehen kann, die gleich oder verschieden sein können, ausgewählt aus der Gruppe bestehend aus Fe, Co, Ni, Cr, Pb, Mn, Zn, Se, Te, Ga, In und As,
C aus einem oder mehreren Elementen bestehen kann, die gleich oder verschieden sein können, ausgewählt aus der Gruppe bestehend aus Alkalimetallen und Tl,
T aus einem oder mehreren Elementen bestehen kann, die gleich oder verschieden sein können, ausgewählt aus der Gruppe bestehend aus Ca, Sr und Ba,
1<m<20, 0≤a≤10, 0≤b≤20, 0≤c≤20, 0≤t≤20, a≤m, b≤m, c≤m, t≤m und x bestimmt wird durch den Grad der Oxidation der anderen Elemente, wobei Sb eine Durchschnittsvalenz höher als +3 und V eine Durchschnittsvalenz niedriger als +5 hat;
BiₙCeₚD_{d}EₑF_{f}Mo₁₂W_{g}O_{y} (2)
wobei
D aus einem oder mehreren Elementen bestehen kann, die gleich oder verschieden sein können, ausgewählt aus der Gruppe bestehend aus Fe, Mn, Pb, Co, Ni, Cu, Sn, P, Cr, Y, Mg, Ca, Sr und Ba,
E aus einem oder mehreren Elementen bestehen kann, die gleich oder verschieden sein können, ausgewählt aus der Gruppe bestehend aus Sb, Ge, As, Se, Te und V,
F aus einem oder mehreren Elementen bestehen kann, die gleich oder verschieden sein können, ausgewählt aus der Gruppe bestehend aus Alkalimetallen, Tl, Ag und Sm,
0,01≤n≤24, 0,01≤p≤24, 0,1≤n+p≤24, 0≤d≤10, 0≤e≤10, 0≤f≤6, 0≤g≤8 und y wird bestimmt durch den Grad der Oxidation der anderen Elemente;
BiₕVᵥLₗMₖT_{q}Oᵣ (3)
wobei
L aus einem oder mehreren Elementen bestehen kann, die gleich oder verschieden sein können, ausgewählt aus der Gruppe bestehend aus K, Cs, Rb und Tl,
M aus einem oder mehreren Elementen bestehen kann, die gleich oder verschieden sein können, ausgewählt aus der Gruppe bestehend aus Mo, W, Cr, Ge oder Sb,
T aus einem oder mehreren Elementen bestehen kann, die gleich oder verschieden sein können, ausgewählt aus der Gruppe bestehend aus Ce, Zn, B, Nb und Ta,
h = 1-25, v = 1-50, 1 = 0-1, vorzugsweise 0-0,2, k = 0,1-20, q = 0-20 und r wird bestimmt durch den Grad der Oxidation der anderen Elemente, (h+v):(l+k+q) = 20:1-1:5, h:v = 1:5-5:1, vorzugsweise 1:3-3:1;
MeₐSb_{b}X_{c}R_{d}QₑO_{f} (4)
worin
Me aus einem oder mehreren Elementen bestehen kann, die gleich oder verschieden sein können, ausgewählt aus der Gruppe bestehend aus Fe, Co, Ta, Ni, Mn, U, Ce, Sn und Cu,
X aus einem oder mehreren Elementen bestehen kann, die gleich oder verschieden sein können, ausgewählt aus der Gruppe bestehend aus V, Mo, W, Nb und Ta,
R aus einem oder mehreren Elementen bestehen kann, die gleich oder verschieden sein können, ausgewählt aus der Gruppe bestehend aus B, P, Bi und Te,
Q aus einem oder mehreren Elementen bestehen kann, die gleich oder verschieden sein können, ausgewählt aus der Gruppe bestehend aus Mg, Ca, Sr, Ba, La, Ti, Zr, Cr, Re, Ru, Os, Rh, Ir, Pd, Pt, Ag, Cd, Al, Ga, In, Tl, Si, B, Ge, As und Se,
a = 10, b = 5-50, c = 0-30, d = 0-10, e = 0-20 und f wird bestimmt durch den Grad der Oxidation der anderen Elemente.

## Revendications

1. Procédé de préparation de (méth)acrylonitrile par ammoxydation d'un mélange d'alcane et d'alcène en C₃ à C₄, caractérisé en ce qu'on utilise un mélange de 50-98% d'alcène et de 2-50% d'alcane et en ce qu'on procède à l'ammoxydation en appliquant une composition de catalyseur qui transforme à la fois l'alcane et l'alcène.

2. Procédé selon la revendication 1, caractérisé en ce que le rapport de mélange est de 65-96% d'alcène et 4-35% d'alcane.

3. Procédé selon l'une quelconque des revendications 1-2, caractérisé en ce que le rapport de mélange est de 85-95% d'alcane et 15-5% d'alcane.

4. Procédé selon l'une quelconque des revendications 1-3, caractérisé en ce qu'on utilise un mélange de propène et de propane.

5. Procédé selon la revendication 3, caractérisé en ce qu'on applique un mélange comportant environ 4% De propane et environ 96% de propène.

6. Procédé selon l'une quelconque des revendications 1-5, caractérisé en ce qu'on utilise une composition de catalyseur qui transforme plus de 80% de l'alcène et plus de 10% de l'alcane.

7. Procédé selon la revendication 6, caractérisé en ce qu'on utilise une composition de catalyseur qui transforme plus de 90% de l'alcène et plus de 50% de l'alcane.

8. Procédé selon l'une quelconque des revendications 1-7, caractérisé en ce qu'on utilise une composition catalyseur formée de deux catalyseurs pour l'ammoxydation.

9. Procédé selon l'une quelconque des revendications 1-8, caractérisé en ce que la composition catalyseur comprend des composés ayant la(les) formule(s) générale(s) (1), (2), (3) et/ou (4) ci-dessous:
VSbₘAₐB_{b}C_{c}TₜOₓ (1)
dans laquelle
A peut consister en un ou plusieurs éléments, qui peuvent être identiques ou différents, choisis dans le groupe comprenant W, Sn, Mo, B, P et Ge,
B peut consister en un où plusieurs éléments, qui peuvent être identiques ou différents, choisis dans le groupe comprenant Fe, Co, Ni, Cr, Pb, Mn, Zn, Se, Te, Ga, In et As,
C peut consister en un ou plusieurs éléments, qui peuvent être identiques ou différents, choisis dans le groupe comprenant les métaux alcalins et Tl,
T peut consister en un ou plusieurs éléments, qui peuvent être identiques ou différents, choisis dans le groupe comprenant Ca, Sr et Ba,
1<m<20, 0≤a≤10, 0≤b≤20, 0≤c≤20, 0≤t≤20, a≤m, b≤m, c≤m, t≤m et x est déterminé par le degré d'oxydation des autres éléments, Sb a une valence moyenne supérieure à +3 et V a une valence moyenne inférieure à +5;
BiₙCeₚD_{d}EₑF_{f}Mo₁₂W_{g}O_{y} (2)
où
D peut consister en un ou plusieurs éléments, qui peuvent être identiques ou différents, choisis dans le groupe comprenant Fe, Mn, Pb, Co, Ni, Cu, Sn, P, Cr, Y, Mg, Ca, Sr et Ba,
E peut consister en un ou plusieurs éléments, qui peuvent être identiques ou différents, choisis dans le groupe comprenant Sb, Ge, As, Se, Te et V,
F peut consister en un ou plusieurs éléments, qui peuvent être identiques ou différents, choisis dans le groupe comprenant les métaux alcalins, Tl ,Ag et Sm, 0,01≤n≤24, 0,01≤p≤24, 0,1≤n+p≤24, 0≤d≤10, 0≤e≤10, 0≤f≤6, 0≤g≤8 et y est déterminé par le degré d'oxydation des autres éléments;
BiₕVᵥLₗMₖT_{q}Oᵣ (3)
où
L peut consister en un ou plusieurs éléments, qui peuvent être identiques ou différents, choisis dans le groupe comprenant K, Cs, Rb et Tl,
M peut consister en un ou plusieurs éléments, qui peuvent être identiques ou différents, choisis dans le groupe comprenant Mo, W, Cr, Ge ou Sb,
T peut consister en un ou plusieurs éléments, qui peuvent être identiques ou différents, choisis dans le groupe comprenant Ce, Zn, B, Nb et Ta,
h = 1-25, v = 1-50, 1 = 0-1, de préférence 0-0,2, k = 0,1-20, q = 0-20 et r est déterminé par le degré d'oxydation des autres éléments, (h+v):(1+k+q) = 20:1-1:5, h:v = 1:5-5:1, de préférence 1:3-3:1;
MeₐSb_{b}X_{c}R_{d}QₑO_{f} (4)
où
Me peut consister en un ou plusieurs éléments, qui peuvent être identiques ou différents, choisis dans le groupe comprenant Fe, Co, Ta, Ni, Mn, U, Ce, Sn et Cu,
X peut consister en un ou plusieurs éléments, qui peuvent être identiques ou différents, choisis dans le groupe comprenant V, Mo, W, Nb et Ta,
R peut consister en un ou plusieurs éléments, qui peuvent être identiques ou différents, choisis dans le groupe comprenant B, P, Bi et Te,
Q peut consister en un ou plusieurs éléments, qui peuvent être identiques ou différents, choisis dans le groupe comprenant Mg, Ca, Sr, Ba, La, Ti, Zr, Cr, Re, Ru, Os, Rh, Ir, Pd, Pt, Ag, Cd, Al, Ga, In, Tl, Si, B, Ge, As et Se,
a = 10, b = 5-50, c = 0-30, d = 0-10, e = 0-20 et f est déterminé par le degré d'oxydation des autres éléments.
